# EUROPEAN PATENT SPECIFICATION

(11) **EP 2 373 371 B1**
(45) Date of publication and mention of the grant of the patent: **20.02.2013**
(21) Application number: 09771607.0
(22) Date of filing: 10.12.2009
(51) Int. Cl.: A61M 1/00, A61M 25/01

(54) **PROPULSION-UNIT AND ROBOT PROVIDED WITH SUCH A PROPULSION-UNIT**
PROPULSIONSGERÄT UND ROBOTER MIT EINEM SOLCHEN PROPULSIONSGERÄT
UNITÉ DE PROPULSION ET ROBOT ÉQUIPÉ DE CETTE UNITÉ DE PROPULSION

(30) Priority: 18.12.2008 NL 2002341
(43) Date of publication of application: 12.10.2011
(73) Proprietor: Technische Universiteit Delft, 2628 CN Delft (NL)
(72) Inventor: BREEDVELD, Paulus, NL-2806 DK Gouda (NL); DODOU, Dimitra, NL-2611 GV Delft (NL)
(74) Representative: Van Breda, Jacobus
(86) International application number: PCT/NL2009/050752
(87) International publication number: WO 2010/071420

(56) References cited:
- US-A- 5 562 601
- US-A1- 2006 241 346

## Description

The invention relates to a propulsion-unit provided with a body and comprising at least one element having an external adhesive layer for providing frictional contact with an object's surface along which the unit in use moves.

Such a propulsion-unit is used for locomotion of an intestine inspection and/or intervention device, which is moveable in a person's colon. Such a propulsion-unit is required to have very moderate dimensions, notably having a maximum diameter of approximately 30 mm or less. The propulsion-unit of the invention may however also show other dimensions (smaller or larger) and may suitably be employed for other purposes or at any other place where propulsion is needed, such as in other confined areas or in spaces that are difficultly accessible such as the digestive system, arteries, cavities in the nose or the brain or other bodily parts of a human or animal. The propulsion-unit of the invention may further be employed in other surroundings than animal or human bodies, such as industrial applications with difficultly accessible spaces such as pipes, tubes or waste-systems as exist in factories or plants for the generation of heat or electricity. Notably heating pipes of boilers are known areas in which it is at times required to carry out inspections for cracks or other adverse situations. Such pipes may pose specific problems due to the many bends that are required to pass in order to carry out a reliable inspection.

In the following description emphasis will be placed on a discussion of the propulsion-unit of the invention with reference to its application as a means to provide locomotion to a colonoscope. The invention is however broader in scope and not restricted to this particular application. Other applications as mentioned above are likewise covered by the instant invention and the appended claims.

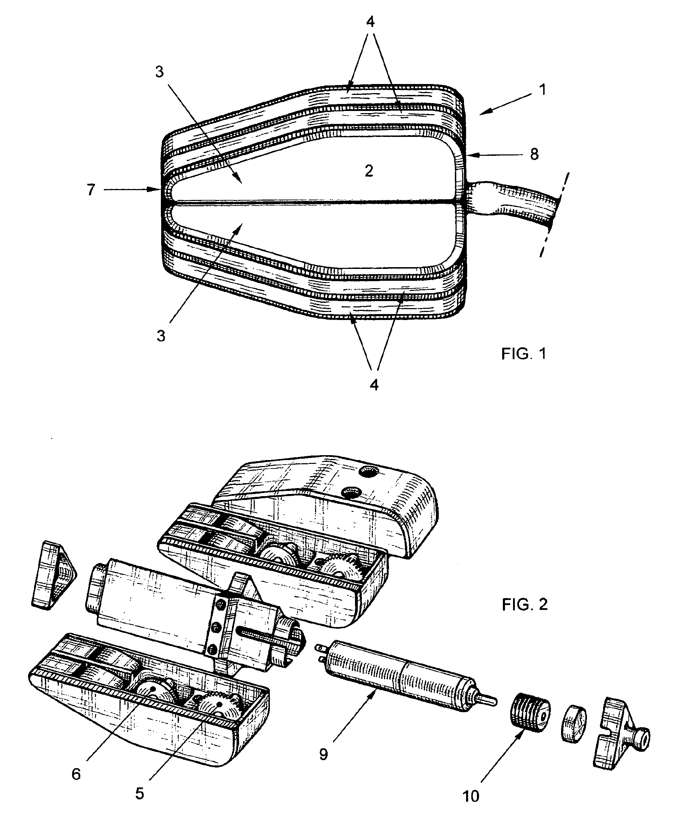

A propulsion-unit in accordance with the preamble is known from the doctoral thesis entitled 'Colonic locomotion' by Dimitra Dodou of September 2006, ISBN-10:90-9020968-9 and ISBN-13:978-90-9020968-5. This doctoral thesis amongst others investigates the application of muco-adhesives and investigates to manipulate friction and to achieve locomotion of a colonoscopic device for movement through the large intestine. Fig. 2.1 of said thesis shows that a muco-adhesive layer is applied at the outer circumference of a series of cylinders that are interconnected by having these cylinders placed on a connecting tube. The cylinders sequentially provide a high frictional contact with the inner wall of the intestine, which is alternated by implementing a periodic reduction of this high frictional contact. Thus, the cylinders with reduced frictional contact may slide through the intestine, and the series of cylinders may move through the intestine by forming a repetitious peristaltic movement.

The application of a muco-adhesive layer on the outside of the moving cylinders provides a solution to the problem which is known from other propulsion-units that are applied for movement in the large intestine, employing balloons that are repeatedly inflated and deflated. Such systems employing balloons are for instance known from US-3,895,637; US-4,676,228; US-4,690,131; US-5,337,732; US-5,398,670; US-5,454,364. The known systems employing balloons suffer from the disadvantage that the friction afforded against the intestine's inner wall is too little to provide a reliable propulsion in said intestine, unless such balloons are inflated to such extend that they cause pain to the concerning person.

To address this problem it is also known to employ means to vacuumize against the intestine's inner wall such as known from US-5,906,591; US-6,309,346 B1.

Other systems to provide traction against the intestine's inner wall are known from US-B-6,648,814 using wheels and the application of belts for this purpose is known from US-6,695,771 B2, US-5,562,601; US-6,071,234; US-6,224,544 B1.

From the above discussion it is clear that there is a long felt need to provide a propulsion-unit to reliably provide traction in the intestines of a person or animal, whereby a swift operation of the unit is desirable. A condition at all times is that damage to the intestines is avoided. Such a reliable propulsion-unit is particularly needed when it is intended to move a colonoscope into a person's or animal's colon so as to inspect said colon. The problem that arises when moving a colonoscope in the colon is that the colon is not straight but shows unexpected turns, which makes it hard to move the colonoscope through the large intestine. At times this may result in the colonoscope getting stuck or damaging the intestine whereby the person or animal that undergoes the inspection may experience severe pain.

The invention as further disclosed in claim 1 is therefore aimed at providing a propulsion-unit which at least in part addresses the above problems and allows for application in combination with a colonoscope or with any other instrument that is required to be propelled, and in general to provide an alternative to existing systems that serve this purpose.

The propulsion-unit and, if applicable, any robot that is provided with such a propulsion-unit is to this end characterized by one or more of the appended claims.

US2006/241346 A1 teaches a propulsion unit provided with a body and comprising at least one element having an external adhesive layer for providing frictional contact with an object's surface along which the unit in use moves, wherein the element that is provided with the external adhesive layer is a supporting layer that supports said adhesive layer and that drive means are provided for moving the supporting layer and the body of the propulsion-unit with respect to each other. The propulsion unit of the invention has the feature that the adhesive layer provided on the supporting layer or supporting layers is a muco-adhesive layer, and that the supporting layer or supporting layers carrying the adhesive layer is provided at regular distances with portions having hydrophilic properties. By this measure it is secured that the supporting layer with the adhesive layer provides the required frictional contact with the object along which the unit is intended to be moved. This is particularly useful as indicated above in the intestinal tract, in particular the colon of a person or animal in which the conditions are such that frictional contact with the wall of the large intestine is difficult to attain. A muco-adhesive layer is very suited for sticking to the wall of the intestines, and with the portions having hydrophilic properties it is possible to control the humidity condition at which the adhesive layer operates. In this manner it is possible to deal with circumstances that may otherwise deteriorate the adhesive properties of the mu-co-adhesive layer.

In order to be able to move the propulsion-unit over a sufficient length, it is desirable that the propulsion-unit is provided with at least one reel for storing the supporting layer.

There may be a reel for storing supporting layer that is being discharged such that in an application wherein the intestinal tract of a person or animal is investigated, the discharged supporting layer is left in the intestinal tract, thereby wasting its environment. It is, however, preferred that there is also a reel for winding up of the supporting layer during operation of the drive means for propelling the unit through the intestinal tract.

The inventors have found that the length of supporting layer that may be stored on a reel may be increased by embodying the reel or reels for the supporting layer such that it has a bearing allowing it to rotate at the body's circumference, as opposed to a construction in which the reel operates in line with the direction of movement of the propulsion-unit. This provides ample storing facility of the supporting layer, which makes the propulsion-unit of the invention very suitable for colonoscopic inspections.

Suitably the propulsion-unit is provided with at least one set of reels, each set comprising a discharge reel and a wind-up reel for a supporting layer. Advantageously, there may be several sets of reels oriented at regular positions around a longitudinal body axis of the propulsion-unit so as to provide evenly distributed forces aimed at the inner wall of the intestines. Each of the (discharge and wind-up) reels may also carry more than one supporting layer, in order to meet desired length and width or diameter requirements of the propulsion-unit.

Suitably further the drive means provide, while the unit is in use, traction to the supporting layer so as to have this supporting layer move as compared to the body of the propulsion-unit.

Preferably, the drive means are drivingly connected to the reel or reels for winding up the supporting layer.

Advantageously the drive means are arranged for selectively driving the respective supporting layers that may be provided at the outer circumference of the unit, at individually defined speeds. This allows that the propulsion-unit has a controllable direction of movement by properly controlling the speeds of said supporting layers.

A preferable embodiment of the propulsion-unit of the invention has the feature that it comprises a housing that is provided with at least one slit through which the supporting layer is guided and that the supporting layer lies adjacent to the housing, or adjacent to parts fixed to the housing.

A very practical embodiment in which the supporting layer after its use is prevented from wasting its environment is embodied with the feature that same has a housing that is provided with at least one frontal slit and at least one backward slit, and that the supporting layer is guided from within the housing through the frontal slit and adjacent to the housing to the backward slit and through said backward slit back into the housing.

It is preferable, particularly in the just-mentioned embodiment, that the at least one reel or the reels of a set of reels for storing the supporting layer or layers is or are located near to the at least one slit or slits in the housing.

The propulsion-unit of the invention may suitably be embodied such that the body equates with the housing. If this is the case, usually the reels for storing the supporting layer or layers release and take up the supporting layer or layers straightly in line with the propulsion direction of the propulsion-unit of the invention.

A most preferred embodiment of the propulsion-unit of the invention is however characterized in that the body is provided in a housing that has frontal and backward slits through which a supporting layer or supporting layers are guided adjacent to the housing, and which supporting layer or supporting layers are in use moved from within the housing through the frontal slit or slits towards the backward slit or slits and back into the housing. Particularly, with this embodiment it is possible to store large amounts of supporting layers, which facilitates the movement of the propulsion-unit over extended length in the intestinal tract, or in the other application areas in which the propulsion-unit may be employed.

The invention will hereinafter be further elucidated with reference to some exemplary embodiments of the propulsion-unit and the robot of the invention and with reference to the drawing.

In the drawing:
- Fig. 1 shows a first embodiment of the propulsion-unit of the invention;
- Fig. 2 shows dismounted parts of the propulsion-unit shown in Fig. 1;
- Fig. 3 shows a second embodiment of the propulsion-unit of the invention;
- Fig. 4 shows interior parts of the propulsion-unit according to Fig. 3 with the housing of the propulsion-unit removed;
- Fig. 5 shows a third embodiment of the propulsion-unit of the invention, whereby the housing of the propulsion-unit is partially removed;
- Fig. 6 shows a robot comprising some propulsion-units in accordance with the invention;
- Fig. 7 and Fig. 8 show two further embodiments of the propulsion-unit of the invention;
- Fig. 9 shows still a further embodiment of the propulsion-unit of the invention.

Wherever in the figures the same reference numerals are applied, these numerals refer to the same parts.

Fig. 1 shows a first embodiment of the propulsion-unit 1 of the invention having a body 2, which also acts as the unit's housing.

The propulsion-unit 1 is embodied with several parts 3 at the outer circumference of which, a film 4 is guided. In this embodiment the supporting layer is a film 4 that supports an adhesive layer facing away from the parts 3 so as to be able to come in contact with a surface of an object along which the unit 1 has to be propelled. Depending on the circumstances instead of a film 4, also use can be made of a caterpillar track, a belt or any other suitable means to support the adhesive layer.

The unit 1 is further provided with drive means 9, 10, part of which is shown in Fig. 2 showing several lose components of the propulsion-unit 1 of Fig. 1.

It will be clear for the artisan that the film 4 is moved from a reel 6 on the inside of the body 2 through a slit at the frontal end 7 (see Fig. 1) of the unit 1 over and adjacent to the housing 2 towards the back end 8 of the unit 1 so as to be wound up by a further reel 5, which is driven by a preferably electric motor 9 that connects to said reel 5 through a worm wheel 10. When there are more films movable at the outer circumference of the housing 2, there may also be a dedicated electric motor for each film 4, which gives the possibility that each film 4 is having a selectively controllable speed for controlling the movement direction of the unit 1.

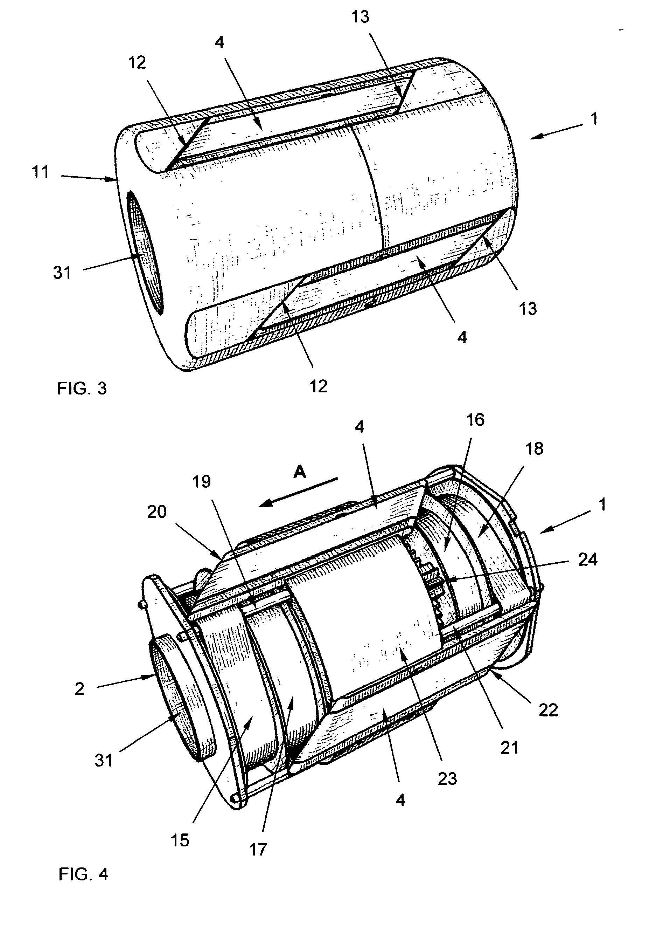

Fig. 3 shows a second embodiment of the propulsion-unit 1 of the invention which is embodied with a housing 11 that is provided with at least one slit 12, 13 through which the film 4 is guided, wherein the film 4 lies adjacent to the housing 11.

Fig. 4 clearly shows that the unit 1 has at least one, and in the shown case two sets of reels 15, 16; 17 18, each set comprising a discharge reel 15, 17 and a wind-up reel 16, 18 for film 4. Also this figure 4 shows clearly that the reels 15, 16; 17, 18 for the film 4 have a bearing allowing it to rotate at the circumference of the body 2. This construction in which the reels 15, 16; 17, 18 are bearingly mounted on the body 2 so as to be able to rotate around said body's circumference for releasing the film 4 from the reels 15, 17 and winding up the film 4 on the reels 16, 18, in a direction perpendicular to the movement direction of the body 2 as indicated with arrow A, arranges that the film 4 may be stored in large quantities on the respective reels 15, 16; 17, 18. Said reels may be arranged to store several films, each of the films on a particular reel leaving and entering the housing through a different slit. The drive means for providing traction to these films may be individually controllable to provide the propulsion-unit with steering capability.

Further there are guiding bars 19, 20, 21, 22 for guiding the film 4 from the earlier-mentioned perpendicular direction to a direction parallel to the movement direction A of the body 2, such that the films come to lie adjacent to the outer circumference of the housing 11. Thus, it is possible that the film is guided from the discharge reels 15, 17 within the housing 11 through the frontal slits 12 in the housing 11 and guided adjacent to said housing 11 to the backward slits 13 and through said backward slits 13 back into the housing 11 so as to be wound up on the wind-up reels 16, 18 that are provided in the housing 11. Suitably, for this purpose the reels 15, 17 from which the films 4 are discharged, are located near the frontal slits 12 whereas the wind-up reels 16, 18 for winding up the used films are located near to the backward slits 13 in the housing 11.

At a central part 23 the body 2 of the propulsion-unit 1 is provided with drive means for driving the film or films 4. For this purpose for instance a transmission gear connecting the wind-up reels 16, 18 to a motor or motors, or other means of driving said wind-up reels 16, 18 may be employed. Said transmission gear forming part of the drive means is indicated with reference numeral 24.

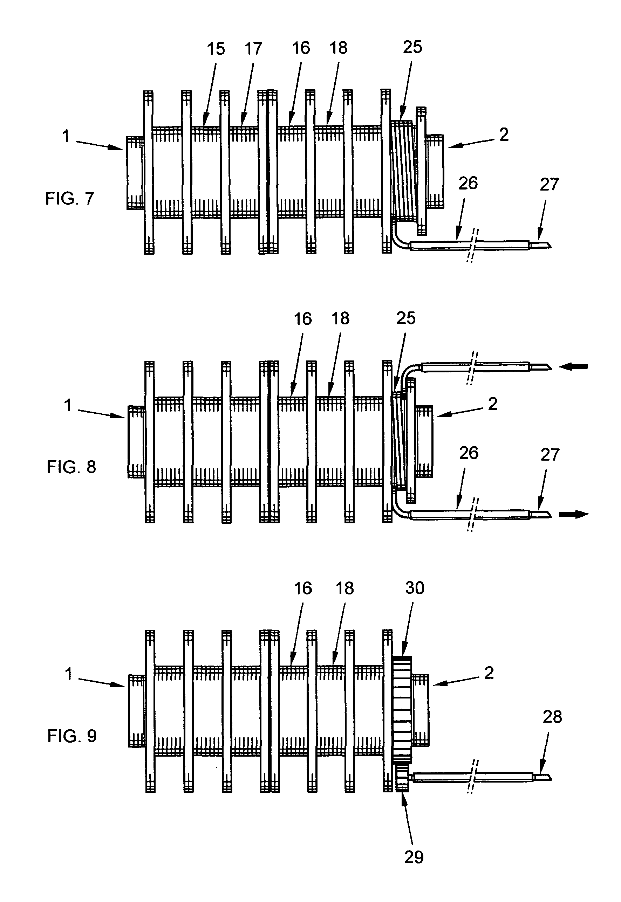

Fig. 7 shows an embodiment of the propulsion-unit 1 of the invention, in which the drive means comprise a pulling cable 25 that is provided in the housing and wound around a suitable part of the body 2 of the propulsion-unit. For clarity purposes the housing is not shown in Fig. 7. The pulling cable 25 connects to wind-up reels 16, 18 either directly or via a transmission gear, and said pulling cable 25 is further guided out of the (not shown) housing through a Bowden-cable 26 to a position 27 distant from the propulsion-unit 1 and its housing so as to be operated therefrom. Instead of a Bowden-cable other suitable guide means that are flexible and axially incompressible can be employed as well, such as a hose, a catheter, a pulling spring. These means may also be employed in plural, as well as there may be plural pulling cables. In the case of plural pulling cables, each cable 25 may connect to a separate wind-up reel for providing individual traction to a film 4, such that the films 4 collectively determine the movement direction of the unit 1.

An embodiment comparable to what is shown in Fig. 7, is shown in Fig. 8. This embodiment comprises either one pulling cable 25 having two free extensions, or two pulling cables 25 making possible that the wind-up reel 16 can be driven in two opposite directions.

Still a further alternative is shown in Fig. 9, in which again the housing is not shown for clarity purposes. In this embodiment the drive means comprise a flexible shaft 28 having a transmission gear 29 near to a corresponding gear 30 on the body 2 that connects to the wind-up reels 16, 18. By rotating the shaft 28 it is possible to wind up the (not shown) film on the wind-up reels 16, 18 and thus provide traction to the propulsion-unit 1 by having the film 4 move along the housing's outer circumference in the longitudinal direction of the unit 1.

Instead of said flexible shaft it is also possible to use a chain of universal joints, a torsionally stiff tube or braided tube or in general a long, flexible torsionally rigid driving body which is rotatable around its body-axis.

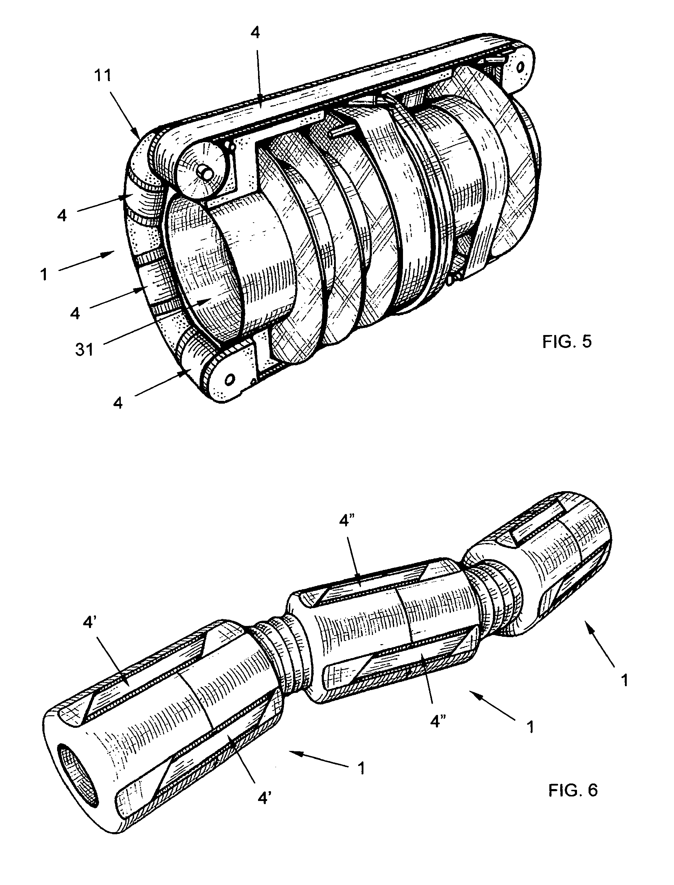

With reference to Fig. 5 a further embodiment of the propulsion-unit 1 of the invention is shown, in which the film or films 4 are guided from a front side to a back side of the housing 11, thereby a larger part of the outer circumference of the housing 11 resulting in improved traction along the inner wall of the object along which the unit 1 is moved.

Finally, Fig. 6 shows a combination of propulsion-units 1 embodied with films 4' and 4'' that are moveable in opposite directions. In this way the robot as shown in Fig. 6 when provided with its own power unit can simply move preprogrammed or with remote control in either direction.

It is further remarked that the propulsion-unit of the invention may be varied in many respects without departing from the gist of the invention as embodied in the appended claims as for instance is illustrated by the embodiment shown in Fig. 5.

The appended claims are therefore not to be considered restricted by the shown embodiments which only serve to resolve any possible ambiguities in respect of the claims.

Amongst the variations that are possible without departing from the scope of protection that merits the invention, the propulsion-unit 1 of the invention may be embodied with a support for the films 4 at the outer circumference of the housing 11 in the form of pressing means for pressing said films away from the housing 11. This may greatly improve the frictional contact between the films 4 of the propulsion-unit with the inner walls of the object's surface along which the unit moves. The pressing means are also beneficial if the propulsion-unit moves in a tube having a varying diameter.

Furthermore, particularly Figs. 3-6 show that the unit 1 may be provided with a central channel 31 through the body 2 for received therein an instrument used for inspecting or treating such as a camera or even a colonoscope, or any other instrument that is considered useful. This instrument may for instance concern a biopsy channel, one or more light sources, one or more analog or digital camera's (preferably remote-controlled), ultrasound probes, or other diagnostic tools, grasping forceps, scissors, biopsy forceps, or other tools for tissue handling and manipulations, a flexible endoscope like a colonoscope, an enteroscope, a sigmoidoscope, a gastroscope, a duodenoscope, a cystoscope, an arthroscope, a larynchoscope, an ureteroscope, a bronchoscope, a fiberscope, or a catheter, a guidewire, a sending and receiving unit for wireless control, a power supply for wireless control, an industrial flexible endoscope such as a boroscope or a video-scope. The propulsion-unit may also effectively be used in combination with an overtube covering one of the above mentioned instruments.

It is further remarked that the film or films 4 that carries the adhesive layer can be suitably provided at regular distances with portions having hydrophilic properties, such as dry paper. This affords the advantage that when the adhesive layer is a muco-adhesive layer which is very suited for sticking to the wall of the intestines, it is possible to control the humidity condition at which the layer operates. In this manner it is possible to deal with circumstances that may otherwise deteriorate the adhesive properties of the muco-adhesive layer.

The muco-adhesive properties of the films can further be promoted by application of holes in the film or films 4 or by providing said films with specific micropatterns.

When the adhesive layer is a muco-adhesive layer, it is further possible to provide the propulsion-unit of the invention with means to release or control the humidity conditions near to the muco-adhesive layer. By these means - for instance water supply means - it is possible to locally neutralize the adhesive properties of said layer, which affords the advantage that directional control of a unit is then possible. The water supply means have for this purpose several selectively controllable outlets near to the muco-adhesive layers which are distributed around the outer circumference of the propulsion-unit.

## Claims

1. Propulsion-unit (1) provided with a body (2) and comprising at least one element having an external adhesive layer for providing frictional contact with an object's surface along which the unit in use moves, wherein the element is a supporting layer (4) that supports said adhesive layer, and that drive means (9, 10) are provided for moving the supporting layer (4) and the body (2) with respect to each other **characterized in that** the adhesive layer provided on the supporting layer or supporting layers (4) is a muco-adhesive layer, and that the supporting layer or supporting layers (4) carrying the adhesive layer is provided at regular distances with portions having hydrophilic properties.

2. Propulsion-unit (1) according to claim 1, **characterized in that** same is provided with at least one reel (5, 6) for storing the supporting layer (4).

3. Propulsion-unit (1) according to claim 1 or 2, **characterized in that** the body (2) houses or supports at least one reel (5, 6) for discharging and/or winding up of the supporting layer (4) during operation of the drive means (9, 10).

4. Propulsion-unit (1) according to any one of claims 1-3, **characterized in that** at least one reel (15, 16, 17, 18) for the supporting layer (4) is provided which reel has a bearing allowing it to rotate at the body's (2) circumference.

5. Propulsion-unit (1) according to any one of claims 1-4, **characterized in that** same is provided with at least one set of reels, each set (15, 16; 17, 18) comprising a discharge reel (15, 17) and a wind-up reel (16, 18) for the supporting layer (4).

6. Propulsion-unit (1) according to any one of the previous claims, **characterized in that** the drive means (9, 10) provide in use traction to the supporting layer (4).

7. Propulsion-unit (1) according to any one of claims 1-6, **characterized in that** the drive means (9, 10) drive in use at least one reel (5) for winding up the supporting layer (4).

8. Propulsion-unit (1) according to any one of claims 1-7, **characterized in that** same has a housing (11) that is provided with at least one slit (12, 13) through which the supporting layer (4) is guided, and that said supporting layer (4) lies adjacent to the housing (11).

9. Propulsion-unit (1) according to any one of claims 1-8, **characterized in that** same has a housing (11) in which at least one reel for the supporting layer (4) is housed, which supporting layer is guided through at least one slit (12, 13) in the housing (11) and adjacent to its outside surface from a frontal portion to a back position of the housing (11).

10. Propulsion-unit (1) according to any one of claims 1-9, **charactarized** in that same has a housing (11) that is provided with at least one frontal slit (12) and at least one backward slit (13), and that the supporting layer (4) is guided from within the housing (11) through the frontal slit (12) and adjacent to the housing (11) to the backward slit (13) and through said backward slit (13) back into the housing (11).

11. Propulsion-unit (1) according to any one of claims 2-10, **characterized in that** the at least one reel or the reels (15, 16; 17 18) of a set of reels for storing the supporting layer (4) is or are located near to the at least one slit or slits (12, 13) in the housing (11).

12. Propulsion-unit (1) according to any one of claims 8-11, **characterized in that** the body (2) is the housing.

13. Propulsion-unit (1) according to any one of claims 1-11, **characterized in that** the body (2) is provided in a housing (11) that has frontal (12) and backward (13) slits through which a supporting layer or supporting layers (4) are guided adjacent to the housing (11), and which supporting layer or supporting layers (4) are in use moved from within the housing (11) through the frontal slit or slits (12) towards the backward slit or slits (13) and back into the housing (11).

14. Propulsion-unit (1) according to any one of the previous claims, **characterized in that** it is provided with drive means for driving the supporting layer or supporting layers. 15. Propulsion-unit (1) according to any one of

15. Propulsion-unit (1) according to any one of claims 2-14, **characterized in that** it is provided with drive means (24) for driving a reel or reels (16, 18) for the supporting layer or supporting layers (4).

16. Propulsion-unit (1) according to any one of claims 1-15, **charaterized** in that there are at least two supporting layers (4) and that the drive means (24) are arranged for selectively driving the supporting layers (4) at individually defined speeds.

17. Propulsion-unit (1) according to any one of the previous claims, **characterized in that** the drive means (24) are supported by the body (2) and/or the housing (11).

18. Propulsion-unit (1) according to any one of the previous claims, **characterized in that** the drive means comprise an electrical motor (9) or motors.

19. Propulsion-unit (1) according to any one of claims 1-16, **characterized in that** the drive means comprise at least one pulling cable (25) provided in the housing and guided from said housing through a flexible and axially incompressible guide tube to a position (27) distant from the housing which guide tube is selected from the group comprising a Bowden-cable (26), a hose, a catheter, a pulling spring.

20. Propulsion-unit (1) according to any one of claims 1-16, **characterized in that** the drive means are selected from the group comprising a flexible shaft, a chain of universal joints, a torsionally stiff tube or braided tube, a long and flexible torsionally rigid driving body (28) for imparting a torsional driving torque to a reel or reels (16, 18) for the supporting layer or supporting layers (4).

21. Propulsion-unit (1) according to any one of claims 14-20, **characterized in that** the reel or reels (15, 16, 17, 18) are bearingly mounted on the body (2) so as to be able to rotate around said body's circumference for discharging and/or winding up the supporting layer or supporting layers (4) in a direction perpendicular to the movement direction (A) of the body (2), and that there are guiding bars (19, 20, 21, 22) for guiding said supporting layer or supporting layers (4) from said perpendicular direction to a direction parallel to the movement direction (A) of the body (2) wherein the supporting layer or supporting layers (4) lie adjacent to the housing's (11) outer circumference.

22. Propulsion-unit (1) according to any one of claims 1-21, **characterized in that** the supporting layer or supporting layers (4) are supported by pressing means for pressing said supporting layer or supporting layers away from said housing (11).

23. Propulsion-unit (1) according to any one of the previous claims, **characterized in that** it is provided with a central channel (31) through said body (2) for receiving therein an instrument selected from the group comprising a biopsy channel, one or more light sources, one or more analog or digital camera's, ultrasound probes or other diagnostic tools, grasping forceps, scissors, biopsy forceps, or other tissue handling or manipulation tools, a flexible endoscope like a colonoscope, an enteroscope, a sigmoidoscope, a gastroscope, a duodenoscope, a cystoscope, an arthroscope, a larynchoscope, an ureteroscope, a bronchoscope, a fiberscope, or a catheter, a guidewire, a sending and receiving unit for wireless control, a power supply for wireless control, an industrial flexible endoscope such as a boroscope or a videoscope.

24. Propulsion-unit (1) according to any one of the previous claims, **characterized in that** the supporting layer is selected from the group comprising a film (4), a caterpillar track, a belt.

25. Propulsion-unit (1) according to any one of the previous claims, **characterized in that** there are water supply means to neutralize the adhesive properties of the muco-adhesive layer.

26. Propulsion-unit (1) according to claim 25, **characterized in that** the water supply means are selectively controllable for providing directional control to the unit.

27. Robot provided with at least one propulsion-unit (1) according to any one of claims 1-26.

28. Robot according to claim 27, **characterized in that** it comprises at least two propulsion-units (1), wherein the two propulsion-units (1) have supporting layers (4', 4") that are moveable in opposite directions.

## Patentansprüche

1. Vortriebseinheit (1), die mit einem Korpus (2) versehen ist und mindestens ein Element umfasst, das eine externe Klebeschicht zum Erzeugen eines Reibungskontakts mit der Oberfläche eines Gegenstandes aufweist, entlang der sich die Einheit während des Gebrauchs bewegt, wobei das Element eine Stützschicht (4) ist, welche die Klebeschicht stützt, und wobei ein Antriebsmittel (9, 10) vorhanden ist, um die Stützschicht (4) und den Korpus (2) relativ zueinander zu bewegen, **dadurch gekennzeichnet, dass** die Klebeschicht, die auf der Stützschicht oder den Stützschichten (4) angeordnet sind, eine Muko-Klebeschicht ist, und dass die Stützschicht oder die Stützschichten (4), welche die Klebeschicht tragen, in regelmäßigen Abständen angeordnet sind, wobei Abschnitte hydrophile Eigenschaften aufweisen.

2. Vortriebseinheit (1) nach Anspruch 1, **dadurch gekennzeichnet, dass** diese mit mindestens einer Haspel (5, 6) zum Speichern der Stützschicht (4) versehen ist.

3. Vortriebseinheit (1) nach Anspruch 1 oder 2, **dadurch gekennzeichnet, dass** der Korpus (2) mindestens eine Haspel (5, 6) aufnimmt oder stützt, um die Stützschicht (4) während des Betriebes des Antriebsmittels (9, 10) auszugeben und/oder aufzuwickeln.

4. Vortriebseinheit (1) nach einem der Ansprüche 1-3, **dadurch gekennzeichnet, dass** mindestens eine Haspel (15, 16, 17, 18) für die Stützschicht (4) vorhanden ist, wobei diese Haspel ein Lager aufweist, das es ihr ermöglicht, sich am Umfang des Korpus (2) zu drehen.

5. Vortriebseinheit (1) nach einem der Ansprüche 1-4, **dadurch gekennzeichnet, dass** diese mit mindestens einem Satz Haspeln versehen ist, wobei jeder Satz (15, 16; 17, 18) eine Abgabehaspel (15, 17) und eine Aufwickelhaspel (16, 18) für die Stützschicht (4) umfasst.

6. Vortriebseinheit (1) nach einem der vorangehenden Ansprüche, **dadurch gekennzeichnet, dass** das Antriebsmittel (9, 10) während des Gebrauchs eine Zugkraft an die Stützschicht (4) anlegt.

7. Vortriebseinheit (1) nach einem der Ansprüche 1-6, **dadurch gekennzeichnet, dass** das Antriebsmittel (9, 10) während des Gebrauchs mindestens eine Haspel (5) antreibt, um die Stützschicht (4) aufzuwickeln.

8. Vortriebseinheit (1) nach einem der Ansprüche 1-7, **dadurch gekennzeichnet, dass** diese ein Gehäuse (11) aufweist, das mit mindestens einem Schlitz (12, 13) versehen ist, durch welche die Stützschicht (4) hindurchgeführt wird, und dass die Stützschicht (4) neben dem Gehäuse (11) liegt.

9. Vortriebseinheit (1) nach einem der Ansprüche 1-8, **dadurch gekennzeichnet, dass** diese ein Gehäuse (11) aufweist, in dem mindestens eine Haspel für die Stützschicht (4) aufgenommen ist, wobei diese Stützschicht durch mindestens einen Schlitz (12, 13) in dem Gehäuse (11) und neben seiner Außenfläche von einem vorderseitigen Abschnitt zu einer hinteren Position des Gehäuses (11) hindurchgeführt wird.

10. Vortriebseinheit (1) nach einem der Ansprüche 1-9, **dadurch gekennzeichnet, dass** diese ein Gehäuse (11) aufweist, das mit mindestens einem vorderseitigen Schlitz (12) und mindestens einem rückseitigen Schlitz (13) versehen ist, und dass die Stützschicht (4) von innerhalb des Gehäuses (11) durch den vorderseitigen Schlitz (12) hindurch und neben dem Gehäuse (11) zu dem rückseitigen Schlitz (13) und durch den rückseitigen Schlitz (13) hindurch in das Gehäuse (11) zurückgeführt wird.

11. Vortriebseinheit (1) nach einem der Ansprüche 2-10, **dadurch gekennzeichnet, dass** die mindestens eine Haspel oder die Haspeln (15, 16; 17 18) eines Satzes Haspeln zum Speichern der Stützschicht (4) nahe dem mindestens einen Schlitz oder den Schlitzen (12, 13) in dem Gehäuse (11) ist bzw. angeordnet sind.

12. Vortriebseinheit (1) nach einem der Ansprüche 8-11, **dadurch gekennzeichnet, dass** der Korpus (2) das Gehäuse ist.

13. Vortriebseinheit (1) nach einem der Ansprüche 1-11, **dadurch gekennzeichnet, dass** der Korpus (2) in einem Gehäuse (11) angeordnet ist, das vorderseitige (12) und rückseitige (13) Schlitze aufweist, durch die eine Stützschicht oder Stützschichten (4) neben dem Gehäuse (11) hindurchgeführt werden, und wobei die Stützschicht oder die Stützschichten (4) während des Gebrauchs von innerhalb des Gehäuses (11) durch den oder die vorderseitigen Schlitze (12) hindurch in Richtung des oder der rückseitigen Schlitze (13) und zurück in das Gehäuse (11) geführt werden.

14. Vortriebseinheit (1) nach einem der vorangehenden Ansprüche, **dadurch gekennzeichnet, dass** sie mit einem Antriebsmittel zum Antreiben der Stützschicht oder der Stützschichten versehen ist.

15. Vortriebseinheit (1) nach einem der Ansprüche 2-14, **dadurch gekennzeichnet, dass** sie mit einem Antriebsmittel (24) zum Antreiben einer Haspel oder von Haspeln (16, 18) für die Stützschicht oder die Stützschichten (4) versehen ist.

16. Vortriebseinheit (1) nach einem der Ansprüche 1-15, **dadurch gekennzeichnet, dass** mindestens zwei Stützschichten (4) vorhanden sind und dass das Antriebsmittel (24) dafür ausgelegt ist, die Stützschichten (4) mit individuell definierten Geschwindigkeiten anzutreiben.

17. Vortriebseinheit (1) nach einem der vorangehenden Ansprüche, **dadurch gekennzeichnet, dass** das Antriebsmittel (24) durch den Korpus (2) und/oder das Gehäuse (11) gestützt wird.

18. Vortriebseinheit (1) nach einem der vorangehenden Ansprüche, **dadurch gekennzeichnet, dass** das Antriebsmittel einen oder mehrere Elektromotoren (9) umfasst.

19. Vortriebseinheit (1) nach einem der Ansprüche 1-16, **dadurch gekennzeichnet, dass** das Antriebsmittel mindestens ein Zugseil (25) umfasst, das in dem Gehäuse angeordnet ist und von dem Gehäuse durch eine flexible und axial nicht zusammendrückbare Führungsröhre hindurch zu einer Position (27) geführt wird, die von dem Gehäuse entfernt liegt, wobei die Führungsröhre aus folgender Gruppe ausgewählt ist: ein Bowdenzug (26), ein Schlauch, ein Katheter und eine Zugfeder.

20. Vortriebseinheit (1) nach einem der Ansprüche 1-16, **dadurch gekennzeichnet, dass** das Antriebsmittel aus folgender Gruppe ausgewählt ist: eine flexible Welle, eine Aneinanderreihung von Kreuzgelenken, eine torsionssteife Röhre oder eine geflochtene Röhre und ein langer und flexibler, torsionsstarrer Antriebskorpus (28), um ein torsionales Antriebsdrehmoment zu einer Haspel oder mehreren Haspeln (16, 18) für die Stützschicht oder die Stützschichten (4) zu übertragen.

21. Vortriebseinheit (1) nach einem der Ansprüche 14-20, **dadurch gekennzeichnet, dass** die Haspel oder die Haspeln (15, 16, 17, 18) derart an dem Korpus (2) gelagert montiert sind, dass sie sich um den Umfang des Korpus herum drehen können, um die Stützschicht oder die Stützschichten (4) in einer Richtung senkrecht zu der Bewegungsrichtung (A) des Korpus (2) abzugeben und/oder aufzuwickeln, und dass Führungsstäbe (19, 20, 21, 22) vorhanden sind, um die Stützschicht oder die Stützschichten (4) aus der senkrechten Richtung in eine Richtung parallel zur Bewegungsrichtung (A) des Korpus (2) zu führen, wobei die Stützschicht oder die Stützschichten (4) neben dem Außenumfang des Gehäuses (11) liegen.

22. Vortriebseinheit (1) nach einem der Ansprüche 1-21, **dadurch gekennzeichnet, dass** die Stützschicht oder die Stützschichten (4) durch ein Drückmittel gestützt werden, das die Stützschicht oder die Stützschichten von dem Gehäuse (11) fort drückt.

23. Vortriebseinheit (1) nach einem der vorangehenden Ansprüche, **dadurch gekennzeichnet, dass** sie mit einem zentralen Kanal (31) versehen ist, der durch den Korpus (2) hindurch verläuft, um darin ein Instrument aufzunehmen, das aus folgender Gruppe ausgewählt ist: ein Biopsiekanal, eine oder mehrere Lichtquellen, eine oder mehrere analoge oder digitale Kameras, Ultraschallsonden oder andere Diagnosewerkzeuge, eine Greifpinzette, eine Schere, eine Biopsiepinzette oder sonstige Gewebehandhabungs- oder - manipulationswerkzeuge, ein flexibles Endoskop wie ein Kolonoskop, ein Enteroskop, eine Sigmoidoskop, ein Gastroskop, ein Duodenoskop, ein Zystoskop, ein Arthroskop, ein Larynchoskop, ein Ureteroskop, ein Bronchoskop, ein Fiberskop oder ein Katheter, ein Führungsdraht, eine Sende- und Empfangseinheit für eine Drahtlossteuerung, eine Stromversorgung für eine Drahtlossteuerung, ein industrielles flexibles Endoskop wie zum Beispiel ein Boroskop oder ein Videoskop.

24. Vortriebseinheit (1) nach einem der vorangehenden Ansprüche, **dadurch gekennzeichnet, dass** die Stützschicht aus folgender Gruppe ausgewählt ist: ein Film (4), eine Gleiskette, ein Band.

25. Vortriebseinheit (1) nach einem der vorangehenden Ansprüche, **dadurch gekennzeichnet, dass** Wasserzuleitungsmittel vorhanden sind, um die Klebeeigenschaften der Muko-Klebeschicht zu neutralisieren.

26. Vortriebseinheit (1) nach Anspruch 25, **dadurch gekennzeichnet, dass** die Wasserzuleitungsmittel selektiv steuerbar sind, um die Richtung der Einheit zu steuern.

27. Roboter, der mit mindestens einer Vortriebseinheit (1) nach einem der Ansprüche 1-26 ausgestattet ist.

28. Roboter nach Anspruch 27, **dadurch gekennzeichnet, dass** er mindestens zwei Vortriebseinheiten (1) umfasst, wobei die zwei Vortriebseinheiten (1) Stützschichten (4', 4") aufweisen, die in entgegengesetzten Richtungen bewegt werden können.

## Revendications

1. Unité de propulsion (1) prévue avec un corps (2) et comprenant au moins un élément ayant une couche adhésive externe pour fournir le contact de friction avec une surface d'objet le long de laquelle l'unité se déplace, à l'usage, dans laquelle l'élément est une couche de support (4) qui supporte ladite couche adhésive, et en ce que des moyens d'entraînement (9, 10) sont prévus pour déplacer la couche de support (4) et le corps (2) l'un par rapport à l'autre, **caractérisée en ce que** la couche adhésive prévue sur la couche de support ou les couches de support (4) est une couche muco-adhésive, et **en ce que** la couche de support ou les couches de support (4) portant la couche adhésive est (sont) prévue(s) à des distances régulières avec des parties ayant des propriétés hydrophiles.

2. Unité de propulsion (1) selon la revendication 1, **caractérisée en ce que** cette dernière est prévue avec au moins une bobine (5, 6) pour stocker la couche de support (4).

3. Unité de propulsion (1) selon la revendication 1 ou 2, **caractérisée en ce que** le corps (2) loge ou supporte au moins une bobine (5, 6) pour décharger et/ou enrouler la couche de support (4) durant le fonctionnement des moyens d'entraînement (9, 10).

4. Unité de propulsion (1) selon l'une quelconque des revendications 1 à 3, **caractérisée en ce que** l'on prévoit au moins une bobine (15, 16, 17, 18) pour la couche de support (4), laquelle bobine a un palier lui permettant de tourner sur la circonférence du corps (2).

5. Unité de propulsion (1) selon l'une quelconque des revendications 1 à 4, **caractérisée en ce que** cette dernière est prévue avec au moins un ensemble de bobines, chaque ensemble (15, 16 ; 17, 18) comprenant une bobine de décharge (15, 17) et une bobine d'enroulement (16, 18) pour la couche de support (4).

6. Unité de propulsion (1) selon l'une quelconque des revendications précédentes, **caractérisée en ce que** les moyens d'entraînement (9, 10) fournissent, à l'usage, la traction pour la couche de support (4).

7. Unité de propulsion (1) selon l'une quelconque des revendications 1 à 6, **caractérisée en ce que** les moyens d'entraînement (9, 10) entraînent, à l'usage, au moins une bobine (5) pour enrouler la couche de support (4).

8. Unité de propulsion (1) selon l'une quelconque des revendications 1 à 7, **caractérisée en ce que** cette dernière a un boîtier (11) qui est doté d'au moins une fente (12, 13) à travers laquelle est guidée la couche de support (4), et **en ce que** ladite couche de support (4) est adjacente au boîtier (11).

9. Unité de propulsion (1) selon l'une quelconque des revendications 1 à 8, **caractérisée en ce que** cette dernière a un boîtier (11) dans lequel est logée au moins une bobine pour la couche de support (4), laquelle couche de support est guidée à travers au moins une fente (12, 13) dans le boîtier (11) et de manière adjacente à sa surface extérieure, d'une partie avant à une position arrière du boîtier (11).

10. Unité de propulsion (1) selon l'une quelconque des revendications 1 à 9, **caractérisée en ce que** cette dernière a un boîtier (11) qui est prévu avec au moins une fente avant (12) et au moins une fente arrière (13), et **en ce que** la couche de support (4) est guidée depuis l'intérieur du boîtier (11) en passant par la fente avant (12) et de manière adjacente au boîtier (11), jusqu'à la fente arrière (13) et passe à travers ladite fente arrière (13) pour revenir dans le boîtier (11).

11. Unité de propulsion (1) selon l'une quelconque des revendications 2 à 10, **caractérisée en ce que** la au moins une bobine ou les bobines (15, 16 ; 17, 18) d'un ensemble de bobines pour stocker la couche de support (4) est ou sont positionnée(s) à proximité de la au moins une fente ou des fentes (12, 13) dans le boîtier (11).

12. Unité de propulsion (1) selon l'une quelconque des revendications 8 à 11, **caractérisée en ce que** le corps (2) est le boîtier.

13. Unité de propulsion (1) selon l'une quelconque des revendications 1 à 11, **caractérisée en ce que** le corps (2) est prévu dans un boîtier (11) qui a des fentes avant (12) et arrière (13) à travers lesquelles une couche de support ou des couches de support (4) est (sont) guidée(s) de manière adjacente au boîtier (11), et laquelle couche de support ou lesquelles couches de support (4) passe(nt), à l'usage, de l'intérieur du boîtier (11) par la fente ou les fentes avant (12) vers la fente ou les fentes arrière (13) et reviennent dans le boîtier (11).

14. Unité de propulsion (1) selon l'une quelconque des revendications précédentes, **caractérisée en ce qu'**elle est prévue avec des moyens d'entraînement pour entraîner la couche de support ou les couches de support.

15. Unité de propulsion (1) selon l'une quelconque des revendications 2 à 14, **caractérisée en ce qu'**elle est prévue avec des moyens d'entraînement (24) pour entraîner une bobine ou des bobines (16, 18) pour la couche de support ou les couches de support (4).

16. Unité de propulsion (1) selon l'une quelconque des revendications 1 à 15, **caractérisée en ce qu'**il y a au moins deux couches de support (4) et **en ce que** les moyens d'entraînement (24) sont agencés pour entraîner sélectivement les couches de support (4) à des vitesses définies individuellement.

17. Unité de propulsion (1) selon l'une quelconque des revendications précédentes, **caractérisée en ce que** les moyens d'entraînement (24) sont supportés par le corps (2) et/ou le boîtier (11).

18. Unité de propulsion (1) selon l'une quelconque des revendications précédentes, **caractérisée en ce que** les moyens d'entraînement comprennent un moteur (9) ou des moteurs électrique(s).

19. Unité de propulsion (1) selon l'une quelconque des revendications 1 à 16, **caractérisée en ce que** les moyens d'entraînement comprennent au moins un câble de traction (25) prévu dans le boîtier et guidé à partir dudit boîtier par un tube de guidage souple et axialement incompressible jusqu'à une position (27) distante du boîtier, lequel tube de guidage est choisi dans le groupe comprenant un câble Bowden (26), un tuyau flexible, un cathéter, un ressort de traction.

20. Unité de propulsion (1) selon l'une quelconque des revendications 1 à 16, **caractérisée en ce que** les moyens d'entraînement sont choisis dans le groupe comprenant un arbre flexible, une chaîne de joints universels, un tube torsionnellement rigide ou un tube tressé, un corps d'entraînement torsionnellement rigide long et souple (28) pour communiquer un couple d'entraînement de torsion à une bobine ou des bobines (16, 18) pour la couche de support ou les couches de support (4).

21. Unité de propulsion (1) selon l'une quelconque des revendications 14 à 20, **caractérisée en ce que** la bobine ou les bobines (15, 16, 17, 18) est (sont) montée(s) par palier sur le corps (2) afin de pouvoir tourner autour de la circonférence dudit corps pour décharger et/ou enrouler la couche de support ou les couches de support (4) dans une direction perpendiculaire à la direction de déplacement (A) du corps (2), et **en ce qu'**il y a des barres de guidage (19, 20, 21, 22) pour guider ladite couche de support ou les couches de support (4) de ladite direction perpendiculaire à une direction parallèle à la direction de déplacement (A) du corps (2), dans laquelle la couche de support ou les couches de support (4) est (sont) adjacente(s) à la circonférence externe du boîtier (11).

22. Unité de propulsion (1) selon l'une quelconque des revendications 1 à 21, **caractérisée en ce que** la couche de support ou les couches de support (4) est (sont) supportée(s) par des moyens de pression pour comprimer ladite couche de support ou les couches de support à distance dudit boîtier (11).

23. Unité de propulsion (1) selon l'une quelconque des revendications précédentes, **caractérisée en ce qu'**elle est prévue avec un canal central (31) à travers ledit corps (2) pour recevoir à l'intérieur de ce dernier un instrument choisi dans le groupe comprenant un canal opérateur, une ou plusieurs sources de lumière, une ou plusieurs caméras analogiques ou numériques, des sondes à ultrasons ou d'autres outils de diagnostic, des pinces à préhension, des ciseaux, des pinces à biopsie ou d'autres outils de traitement ou de manipulation de tissu, un endoscope souple comme un colonoscope, un
entéroscope, un sigmoïdoscope, un gastroscope, un duodénoscope, un cystoscope, un arthroscope, un larynchoscope, un urétéroscope, un bronchoscope, un fibroscope ou un cathéter, un fil guide, une unité d'envoi et de réception pour une commande sans fil, une alimentation d'énergie pour une commande sans fil, un endoscope souple industriel tel qu'un boroscope ou un vidéoscope.

24. Unité de propulsion (1) selon l'une quelconque des revendications précédentes, **caractérisée en ce que** la couche de support est choisie dans le groupe comprenant un film (4), une chenille, une courroie.

25. Unité de propulsion (1) selon l'une quelconque des revendications précédentes, **caractérisée en ce qu'**il y a des moyens d'alimentation en eau pour neutraliser les propriétés adhésives de la couche muco-adhésive.

26. Unité de propulsion (1) selon la revendication 25, **caractérisée en ce que** les moyens d'alimentation en eau peuvent être sélectivement commandés pour fournir la commande directionnelle de l'unité.

27. Robot prévu avec au moins une unité de propulsion (1) selon l'une quelconque des revendications 1 à 26.

28. Robot selon la revendication 27, **caractérisé en ce qu'**il comprend au moins deux unités de propulsion (1), dans lequel les deux unités de propulsion (1) ont des couches de support (4', 4") qui sont mobiles dans des directions opposées.
